# EUROPEAN PATENT SPECIFICATION

(11) **EP 3 850 631 B1**
(45) Date of publication and mention of the grant of the patent: **25.11.2026**
(21) Application number: 19779277.3
(22) Date of filing: 13.09.2019
(51) Int. Cl.: G16B 20/10, G16B 40/00, C12Q 1/6869

(54) **METHOD AND APPARATUS FOR DETECTING COPY NUMBER VARIATIONS IN A GENOME**
VERFAHREN UND VORRICHTUNG ZUR ERKENNUNG VON KOPIENZAHLVARIATIONEN IN EINEM GENOM
PROCÉDÉ ET APPAREIL DE DÉTECTION DE VARIATIONS DU NOMBRE DE COPIES DANS UN GÉNOME

(30) Priority: 14.09.2018 US 201862731738 P
(43) Date of publication of application: 21.07.2021
(73) Proprietor: The Jackson Laboratory, Bar Harbor, ME 04609 (US)
(72) Inventor: LEE, Wan-Ping, Avon, CT 06001 (US); ZHANG, Chengsheng, Bar Harbor, ME 04609 (US); ZHU, Qihui, Farmington, CT 06032 (US); LEE, Charles, Marlborough, MA 01752 (US)
(74) Representative: Potter Clarkson
(86) International application number: PCT/US2019/051069
(87) International publication number: WO 2020/056302

(56) References cited:
- CN-A- 108 229 099
- US-A1- 2011 177 517
- S. YOON ET AL: "Sensitive and accurate detection of copy number variants using read depth of coverage", GENOME RESEARCH, vol. 19, no. 9, 5 August 2009 (2009-08-05), pages 1586 - 1592, XP055167321, ISSN: 1088-9051, DOI: 10.1101/gr.092981.109
- CHIU R W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, vol. 105, no. 51, 23 December 2008 (2008-12-23), pages 20458 - 20463, XP055284693, ISSN: 0027-8424, DOI: 10.1073/pnas.0810641105

## Description

### RELATED APPLICATIONS

This Application claims the benefit under 35 U.S.C. § 119(e) of U.S. Provisional Application Serial No. 62/731,738, filed September 14, 2018, entitled "METHOD AND APPARATUS FOR DETECTING COPY NUMBER VARIATIONS IN A GENOME."

### BACKGROUND

Copy number variation (CNV) is a phenomenon in which sections of the genome are duplicated or deleted, and may affect a large number of base pairs in the genome. CNVs may cause microdeletion and microduplication syndromes in humans, as well as other genetic disorders such as autism-spectrum disorders.

Conventional molecular cytogenetic methods, such as chromosomal microarray analysis (CMA) and fluorescent in situ hybridization (FISH) are the standard assays for detection of chromosomal aberrations at clinical laboratories. However, next-generation sequencing (NGS) techniques have made whole genome sequencing (WGS) more accessible, and computational methods are needed to analyze WGS-based assays.

CN 108229099 (A) discloses obtaining a gene sequence on a sample chromosome; aligning the gene sequence with a human genome reference sequence, determining a base sequence reads in the sample chromosome that uniquely matches the human genome reference sequence; counting the number of the base sequence reads in each of the observation regions bin, wherein the observation region bin is a plurality of regions bin obtained by dividing the sample chromosomes according to a predetermined windowing condition; determining a hidden Markov model according to the number of base sequence reads in the plurality of observation regions bin; gene copy number variation CNV is determined according to the hidden Markov model, wherein the copy number variation CNV comprises: at least one observation area bin.

YOON SET AL: "Sensitive and accurate detection of copy number variants using read depth of coverage", GENOME RESEARCH, vol. 19, no. 9, 5 August 2009 (2009-08-05), pages 1586-1592 discloses a method for CNV detection using read depth of coverage.

### BRIEF SUMMARY

Some embodiments are directed to a method for detecting copy number variations (CNVs) in a genetic sequence, the method comprising using a processor to perform steps of: scanning the genetic sequence to identify at least one unique genetic region within an at least one autosomal chromosome; dividing the genetic sequence into a plurality of bins, each bin of the plurality of bins comprising a plurality of base pairs of the genetic sequence; calculating a CNV status for each bin of the plurality of bins; and filtering the CNV statuses to identify at least one CNV in the genetic sequence.

Some embodiments are directed to an at least one non-transitory computer-readable storage medium, having computer-readable instructions stored thereon that, when executed by a processor, cause the processor to execute a method to detect CNVs in a genetic sequence. The method comprises scanning the genetic sequence to identify at least one unique genetic region within an at least one autosomal chromosome; dividing the genetic sequence into a plurality of bins, each bin of the plurality of bins comprising a plurality of base pairs of the genetic sequence; calculating a CNV status for each bin of the plurality of bins; and filtering the CNV statuses to identify at least one CNV in the genetic sequence.

Some embodiments are directed to a system for detecting CNVs in a genetic sequence, the system comprising at least one processor operatively connected to a computer-readable memory. The computer-readable memory contains instructions which, when executed by the at least one processor, cause the at least one processor to perform a method comprising steps of scanning the genetic sequence to identify at least one unique genetic region within an at least one autosomal chromosome; dividing the genetic sequence into a plurality of bins, each bin of the plurality of bins comprising a plurality of base pairs of the genetic sequence; calculating a CNV status for each bin of the plurality of bins; and filtering the CNV statuses to identify at least one CNV in the genetic sequence.

In some embodiments, the genetic sequence is a partial genome sequence. In some embodiments, the genetic sequence is a whole genome sequence (WGS).

In some embodiments, the method comprises aligning the genetic sequence with a reference genome.

In some embodiments, identifying an at least one unique genetic region within the at least one autosomal chromosome comprises: determining that each 25 k-mer of the at least one unique genetic regions appears only once within the genetic sequence; and determining that the at least one unique genetic region comprises greater than 20,000 base pairs.

In some embodiments, the method further comprises calculating a read depth for the genetic sequence.

In some embodiments, the method further comprises: calculating a read depth of the at least one autosomal chromosome based on a read depth of the at least one unique genetic region; comparing the read depth of the at least one autosomal chromosome to the read depth of the genetic sequence; and determining whether the genetic sequence comprises an aneuploidy based on the compared read depths.

In some embodiments, calculating a CNV status for each bin of the plurality of bins comprises: calculating a read depth of each bin of the plurality of bins; converting the read depth of each bin of the plurality of bins into a percentile; and converting the percentile into a CNV status.

In some embodiments, converting the read depth to a percentile comprises: dividing the read depth of each bin of the plurality of bins by the number of base pairs in the plurality of base pairs and multiplying by the read depth of the genetic sequence.

In some embodiments, converting the percentile of each bin to a CNV status comprises applying a Hidden Markov Model (HMM) with a Poisson distribution of read depth of the genetic sequence.

In some embodiments, each bin of the plurality of bins comprises 50 base pairs.

In some embodiments, the method further comprises merging one or more bins of the plurality of bins.

In some embodiments, filtering the CNV statuses comprises: dividing the merged bins into a plurality of regions, each region comprising an equal number of base pairs; assigning a uniqueness value to each region; and filtering out regions having a uniqueness value below a threshold value.

In some embodiments, the uniqueness value is calculated by determining a number of unique k-mers in the regions.

Some embodiments are directed to a method of diagnosing a disorder caused by at least one pathogenic CNV. The method comprises using a processor to perform steps of: scanning the genetic sequence to identify at least one unique genetic region within an at least one autosomal chromosome; dividing the genetic sequence into a plurality of bins, each bin of the plurality of bins comprising a plurality of base pairs of the WGS; calculating CNV statuses for each bin of the plurality of bins; and filtering the CNV statuses to identify at least one CNV in the genetic sequence. The method further comprises determining the identified at least one CNV is an at least one pathogenic CNV; and diagnosing a disorder based on the determined at least one pathogenic CNV.

Some embodiments are directed to a method of treating a disorder caused by at least one pathogenic CNV. The method comprises using a processor to perform steps of: scanning the genetic sequence to identify at least one unique genetic region within an at least one autosomal chromosome; dividing the genetic sequence into a plurality of bins, each bin of the plurality of bins comprising a plurality of base pairs of the WGS; calculating CNV statuses for each bin of the plurality of bins; and filtering the CNV statuses to identify at least one CNV in the WGS. The method further comprises: determining the identified at least one CNV is an at least one pathogenic CNV; diagnosing a disorder based on the at least one pathogenic CNV; and administering a treatment to alleviate one or more symptoms of the diagnosed disorder.

In some embodiments, the disorder is one of a selection of: an autism-spectrum disorder, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

In some embodiments, the genetic sequence is a partial genome sequence. In some embodiments, the genetic sequence is a WGS.

In some embodiments, the method comprises aligning the genetic sequence with a reference genome.

In some embodiments, identifying an at least one unique genetic region within the at least one autosomal chromosome comprises: determining that each 25 k-mer of the at least one unique genetic regions appears only once within the genetic sequence; and determining that the at least one unique genetic region comprises greater than 20,000 base pairs.

In some embodiments, the method further comprises calculating a read depth for the genetic sequence.

In some embodiments, the method further comprises: calculating a read depth of the at least one autosomal chromosome based on a read depth of the at least one unique genetic region; comparing the read depth of the at least one autosomal chromosome to the read depth of the genetic sequence; and determining whether the genetic sequence comprises an aneuploidy based on the compared read depths.

In some embodiments, calculating a CNV status for each bin of the plurality of bins comprises: calculating a read depth of each bin of the plurality of bins; converting the read depth of each bin of the plurality of bins into a percentile; and converting the percentile into a CNV status.

In some embodiments, converting the read depth to a percentile comprises: dividing the read depth of each bin of the plurality of bins by the number of base pairs in the plurality of base pairs and multiplying by the read depth of the genetic sequence.

In some embodiments, converting the percentile of each bin to a CNV status comprises applying a Hidden Markov Model (HMM) with a Poisson distribution of read depth of the genetic sequence.

In some embodiments, each bin of the plurality of bins comprises 50 base pairs.

In some embodiments, the method further comprises merging one or more bins of the plurality of bins.

In some embodiments, filtering the CNV statuses comprises: dividing the merged bins into a plurality of regions, each region comprising an equal number of base pairs; assigning a uniqueness value to each region; and filtering out regions having a uniqueness value below a threshold value.

In some embodiments, the uniqueness value is calculated by determining a number of unique k-mers in the regions.

### BRIEF DESCRIPTION OF DRAWINGS

Various aspects and embodiments will be described with reference to the following figures. It should be appreciated that the figures are not necessarily drawn to scale. In the drawings, each identical or nearly identical component that is illustrated in various figures is represented by a like numeral. For purposes of clarity, not every component may be labeled in every drawing.
FIG. 1A depicts, schematically, an illustrative block diagram of a data pipeline, in accordance with some embodiments of the technology described herein;
FIG. 1B depicts, schematically, an illustrative application of a clustering algorithm to a genetic sequence, in accordance with some embodiments of the technology described herein;
FIG. 1C depicts, schematically, an illustrative application of the data pipeline of FIG. 1A to a genetic sequence, in accordance with some embodiments of the technology described herein;
FIG. 2 is a flowchart describing a process of identifying at least one copy number variation (CNV) in a genetic sequence, in accordance with some embodiments of the technology described herein;
FIG. 3 is a flowchart describing a process of diagnosing a disorder caused by at least one CNV in a genetic sequence, in accordance with some embodiments of the technology described herein;
FIG. 4 is a flowchart describing a process of treating a disorder caused by at least one CNV in a genetic sequence, in accordance with some embodiments of the technology described herein;
FIGs. 5A and 5B show a comparison of detected CNV deletions and duplications for 31 samples as identified by a chromosomal microarray (CMA) performed by the Coriell Institute, a CMA performed by The Jackson Laboratory, and whole genome sequences (WGSs) as analyzed by the JAX-CNV algorithm, in accordance with some embodiments of the technology described herein;
FIGs. 6A shows, as a function of CNV size and for both CNV deletions and CNV duplications, the number of unique CNVs detected by JAX-CNV and the number of CNVs both detected by JAX-CNV and CMAs performed by The Jackson Laboratory on 31 samples, in accordance with some embodiments of the technology described herein;
FIG. 6B shows, for each genetic mutation, the number of unique CNVs detected by JAX-CNV and the number of CNVs detected by both JAX-CNV and CMAs performed by The Jackson Laboratory on 31 samples, in accordance with some embodiments of the technology described herein;
FIG. 7A shows CNV detection by, from top to bottom and for a total of 31 samples, CMAs performed by the Coriell Institute, CMAs performed by The Jackson Laboratory, and analysis of WGSs by JAX-CNV for decreasing coverage values;
FIG. 7B shows, as a function of coverage and for CNV deletions, concordance between JAX-CNV and CMAs performed by The Jackson Laboratory on 31 samples, in accordance with some embodiments of the technology described herein;
FIG. 7C shows, as a function of coverage and for CNV duplications, concordance between JAX-CNV and CMAs performed by The Jackson Laboratory on 31 samples, in accordance with some embodiments of the technology described herein;
FIG. 8 depicts, schematically, an illustrative computing device X on which any aspect of the present disclosure may be implemented, in accordance with some embodiments of the technology described herein.

### DETAILED DESCRIPTION

Copy number variations (CNVs) are sections of the genome that are repeated, with different individuals of a population exhibiting different numbers of repeated genomic material. CNVs form from 4.8 to 9.5% of the human genome, and CNVs are thought to play key roles in human evolution, genomic diversity, and disease susceptibility. However, changes to CNVs between individuals can cause microdeletion and microduplication syndromes with symptoms such as developmental and/or intellectual disabilities. These syndromes may include, but are not limited to, autism-spectrum disorders, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

Different technologies have been used in research and clinical laboratories for CNV detection including fluorescence in situ hybridization (FISH), PCR-based assays, chromosomal microarrays (CMAs) and, most recently, next-generation sequencing (NGS). CMAs are currently used as first-tier diagnostic tests for patients with unexplained developmental delay or intellectual disabilities, autism spectrum disorders, and congenital anomalies. However, CMAs may be costly to perform and are limited in resolution by the number of probes used during the array.

Over the past decade, advances in NGS technologies have brought unprecedented improvements in throughput, speed, and cost of DNA sequencing. These improvements make whole genome sequencing (WGS) feasible for broad use in research and clinical diagnosis with its ability to precisely detect many types of genetic variations. Besides, the advancement of NGS, the rapid development of bioinformatics tools have made analyzing NGS results feasible in clinical laboratories. Although several WGS-based CNV calling algorithms have been developed, none of them are widely accepted for use in a clinical setting because the false positive and false negative rates are often high (e.g., above 5%), making detection of true pathogenic CNVs difficult in a clinical setting.

The inventors have recognized and appreciated that clinical settings are lacking robust computational methods for detecting, accurately and efficiently, CNVs from NGS results. Accordingly, systems and methods are presented herein for detecting CNVs in genetic sequences, including partial genetic sequences (PGS) or whole genetic sequences (WGS).

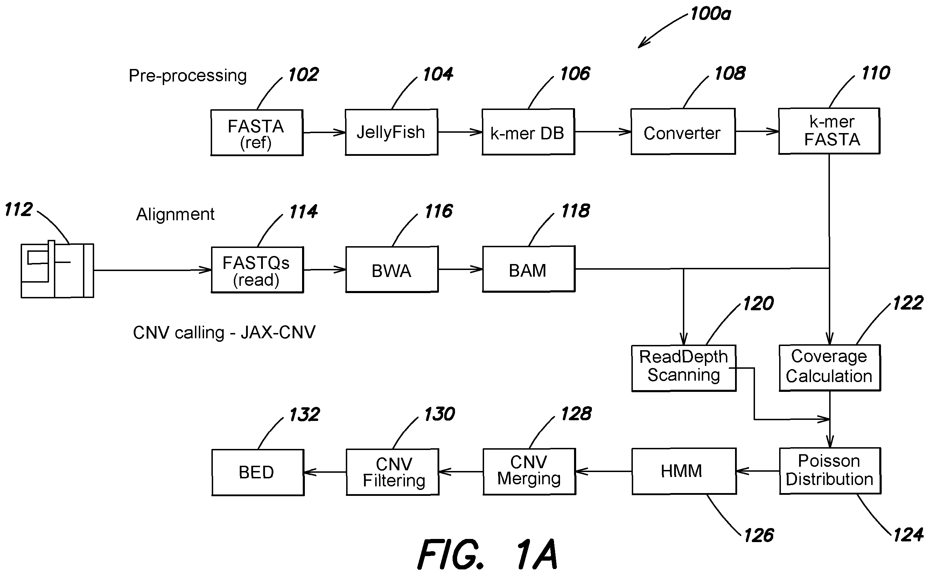

FIG. 1A shows a schematic of a data pipeline 100 configured to call CNVs from a genetic sequence, in accordance with some embodiments of the technology described herein. In some embodiments, the data pipeline 100 may be implemented by hardware (e.g., using an ASIC, an FPGA, or any other suitable circuitry), software (e.g., by executing the software using a computer processor), or any suitable combination thereof.

Pre-processing of a reference genome (e.g., GRCh19 or GRCh38) may occur prior to calling CNVs in a genetic sequence of interest. Pre-processing may occur before every instance of calling CNVs, or only once per reference genome. Pre-processing of a reference genome may comprise reading a reference genome file 102 in a FASTA ("Fast-All") file format, wherein a genetic sequence may be represented in a text-based format using single-letter codes.

In step 104, a calculation of the counts of each k-mer within the genetic sequence of the reference genome may be performed. A k-mer is a substring of a genetic sequence of length k. For example, k may be 25 base pairs (herein, "bp"), though any appropriate value of k may be used. The calculation may be performed by an algorithm such as JELLYFISH (e.g., JELLYFISH v2.2.6). The algorithm may output a k-mer database 106 (herein, "k-mer DB"), in a binary format containing each k-mer string and the number of times it has appeared in the genetic sequence.

In some embodiments, the k-mer DB 106 may be, in step 108, converted to a k-mer FASTA file 110. The k-mer FASTA file 110 may contain the log₂ of the number of times each k-mer has appeared in the genetic sequence. For example, if a k-mer in the k-mer DB 106 appears only once in the genome, the corresponding entry in the k-mer FASTA file 110 is log₂(1) = 0. The entries of the k-mer FASTA file 110 may further be converted to an ASCII code prior to usage in calling CNVs.

Prior to starting the algorithm to call CNVs, the genetic sequence data may be obtained and processed, in accordance with some embodiments. The genetic sequence data may be obtained from, for example, a next-generation sequencing system 112 or any other suitable sequencing method. The genetic sequence data may represent, for example, a partial genetic sequence (PGS) or a whole genome sequence (WGS). The genetic sequence data may be obtained in a FASTQ file 114.

In some embodiments, the FASTQ file may be checked for quality control and/or aligned against the reference genome in step 116. Quality control may be performed by, for example, FASTQC (e.g., FASTQC v0.11.5, not pictured). Alignment of the genetic sequence with the reference genome may be performed by a sequence aligning algorithm, such as, for example BWA-MEM (e.g., BWA-MEM v0.7.15). The alignment results of step 116 may be sorted by sequence coordinates using, for example, SAMTOOLS. A binary file 118 (e.g., a BAM file) containing sequence alignment data in a binary format may be generated by the algorithm of step 116. The binary file 118 may be input to the CNV calling routine (herein, "JAX-CNV").

Results of pre-processing of the reference genome and alignment of the genetic sequence data may next be sent to JAX-CNV, in accordance with some embodiments described herein. A first step of JAX-CNV may be a read depth calculation ("coverage" calculation), performed in step 120, wherein the number of times a specific nucleotide appears in the sequencing results is calculated. A read depth may be calculated for each autosomal chromosome based on one or more unique genetic regions in the chromosome (e.g., 20 unique genetic regions). The k-mer FASTA file 110 and/or BAM file 118 may be scanned to determine unique genetic regions within each autosomal chromosome. A genetic region may be considered unique when each k-mer within the region appears only once and the size of the region is larger than 20 Kb (e.g., 20,000 base pairs). The read depth of each autosomal chromosome may be calculated as an average of the read depths calculated for each base pair of each unique region.

A read depth may then be calculated for the entire sequence of the sample, in some embodiments. An interquartile range may be applied to filter outlier read depth values, and an overall read depth of the genetic sequence may be calculated based on an average of the read depths for all autosomal chromosomes. Comparing the read depth of each chromosome with the read depth of the genetic sequence may detect aneuploidies in the genetic sequence.

In some embodiments, the BAM file 118 may then be divided into bins comprising a same number of base pairs. In some embodiments, the bins may comprise 50 base pairs. A read depth calculation may then be performed in step 122 to calculate a read depth of each bin. The read depth may be further converted to a percentile from 0% to 180%, with 50% representing a baseline read depth. For example, if the read depth of the genetic sequence is 50, and a read depth of a bin is 100, the percentile of the bin will be 100% (100*50%/50).

In steps 124 and 126, a hidden Markov model (HMM) with a Poisson distribution of read depth may be applied to the percentile values, in accordance with some embodiments described herein. The hidden Markov model may convert the percentile of each bin to one of five CNV statuses: CN=0 (deletion), CN=1 (deletion), CN=2 (normal), CN=3 (duplication) and CN>3 (duplication).

In some embodiments, where a bin size is set to a small value (e.g., 50 base pairs), noise may occur in the assigned CNV statuses. Using larger bin sizes may decrease noise but also may decrease sensitivity to small CNVs. Therefore, merging adjacent CNVs in step 128 may mitigate noise in CNV statuses, according to some embodiments described herein. If the CNV status' length is shorter than 5Kb, the status may be merged with a neighboring status. This merging step may cause the resolution of JAX-CNV to be 5Kb.

In some instances, the CNV status merging may merge regions including too many different statuses. To prevent this, if the original status of the region is assigned to less than 80% of the length of the merged region of the sequence, the CNV status merging will stop and reinstate the original statuses and genetic regions. After recognition of a complex region and the cease of merging, the CNV statuses may then sorted by their respective sequence lengths. From the longest to the shortest, each CNV status may scan other statuses downstream and upstream for further merging.

Candidate CNVs may then be generated by filtering the CNV statuses in step 130, in accordance with some embodiments described herein. Each CNV status region may be divided into ten bins of equal length. Each bin may be assigned a uniqueness value corresponding to number of k-mers in the bin which are unique (e.g., only appear once within the genetic sequence). The bins may be sequentially filtered if their uniqueness values are below a threshold value (e.g., if the percentage of unique k-mers is below 60%, though any suitable threshold may be used).

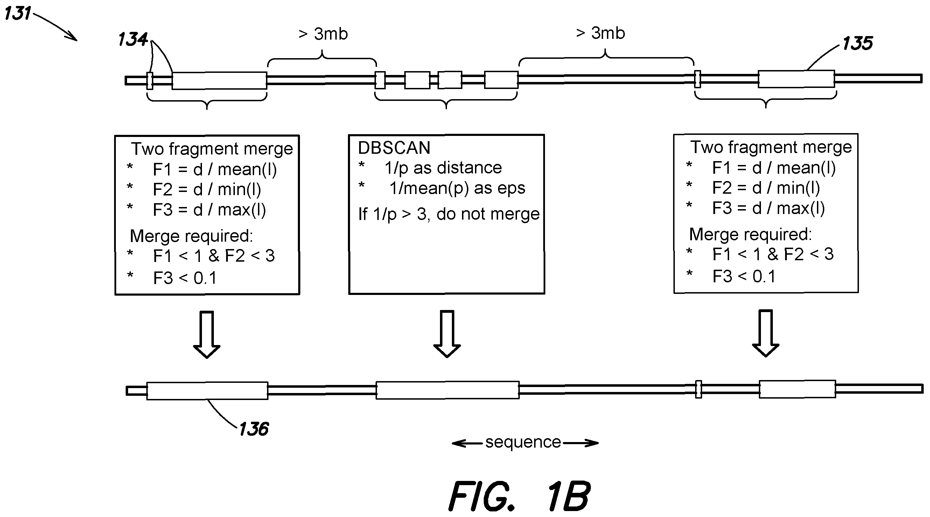

A clustering algorithm (not shown) may be applied after filtering to further cluster the candidate CNV fragments, in some embodiments. For example, a density-based spatial clustering of application with noise (DBSCAN) algorithm 131 may be applied, as is further described in connection with FIG. 1B. The remaining candidate CNV fragments 134 may be sorted based on their positions within the genetic sequence. Then, the CNV fragments 134 may be separated into different raw clusters 135 based on two conditions: a) the distances between any two continuous CNV fragments 134 include fewer than 3,000,000 base pairs; or b) the type (e.g. deletion, duplication) of all fragments located in the raw cluster region are the same. Next, for each raw cluster 135, the distance, d, between every continuous fragment pair fᵢ and fᵢ₊₁ may be calculated as d_{i,i+1}=(eᵢ₊₁-sᵢ)/(lᵢ+lᵢ₊₁), where eᵢ₊₁ is the end position of fᵢ₊₁, sᵢ is the start position of fᵢ, and lᵢ and lᵢ₊₁ are the length of fᵢ and fᵢ₊₁. The mean distance of the raw cluster 135 may also be calculated as dₘₑₐₙ = (E-S)/i = 1Nlᵢ, where E is the end position of the raw cluster, S is the start position of the raw cluster, and N is the number of fragments in the raw cluster.

To overcome the cluster bias on the raw clusters with small and sparse fragments, the distance of a continuous fragment pair may be set as d>3 and the distance of a discontinuous fragment pair may be set as dₘₑₐₙ₊₁. Finally, the DBSCAN function (e.g., the DBSCAN R package) may be applied to the distance matrix of each raw cluster with parameters eps = dₘₑₐₙ and minPts = 2 to obtain clusters. Afterwards, the distance matrix and dₘₑₐₙ may be updated, and DBSCAN may be applied iteratively until the cluster results reach a steady state.

For the raw clusters with only two CNV fragments (denoted as f₁ and f₂, where the sequence position of f₁ is smaller than that of f₂), which cannot be clustered by DBSCAN, three variables may be calculated: y₁=(s₂-e₁) / mean(l₁,l₂), y₂=(s₂-e₁) / min(l₁,l₂), and y3=(s₂-e₁) / max(l₁,l₂). The fragments f₁ and f₂ may be clustered when one of the following two conditions is satisfied: a) y₁<1 and y₂<3; or b) y₃<0.1. Each final cluster 136 may include a CNV and its type (e.g., duplication, deletion). The type of the final clusters 136 may be determined by the CNV type of the fragments 134 in the corresponding raw cluster 135. CNVs may be output in a BED file 132 when the remaining regions of the genetic sequence are larger than 45Kb.

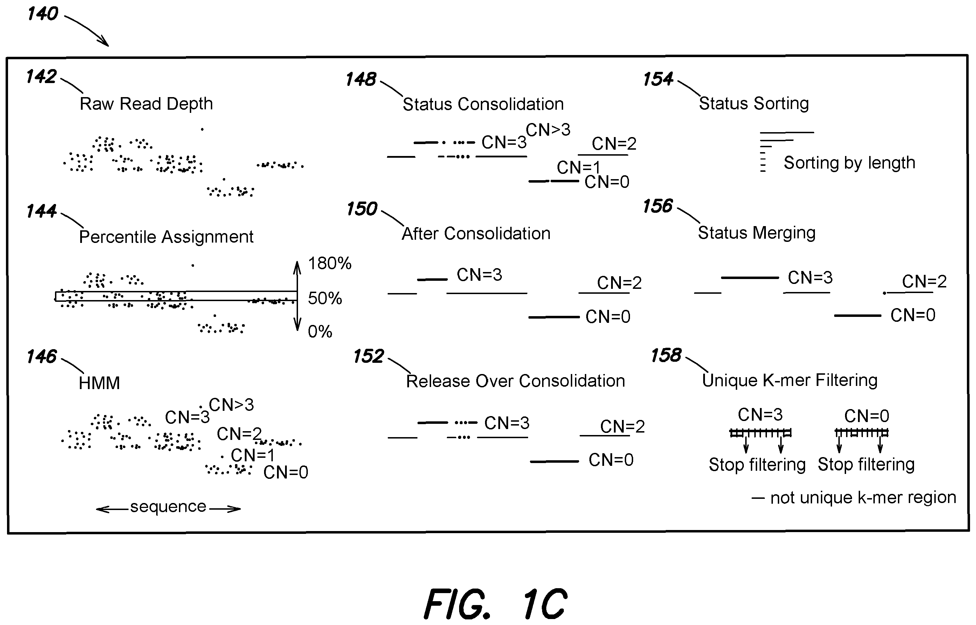

FIG. 1C shows an alternative schematic representation of a JAX-CNV pipeline 140 configured to call CNVs from genetic sequence data, in accordance with some embodiments of the technology described herein. FIC. 1C may show the transformations applied by steps of the data pipeline 100 of FIG. 1A to the input genetic sequence data. In some embodiments, the JAX-CNV pipeline 140 may be implemented by hardware (e.g., using an ASIC, an FPGA, or any other suitable circuitry), software (e.g., by executing the software using a computer processor), or any suitable combination thereof. The horizontal axis of FIG. 1C represents the length of the genetic sequence from first base pair to last base pair of the genetic sequence.

In some embodiments, the BAM file 118 may then be divided into bins comprising a same number of base pairs and a read depth for each bin may be calculated, as shown in step 142. The read depth of each bin may be further converted to a percentile from 0% to 180%, with 50% representing a baseline read depth, as shown in step 144. For example, if the read depth of the genetic sequence is 50, and a read depth of a bin is 100, the percentile of the bin will be 100% (100*50%/50). Steps 142 and 144 may correspond to step 122 of FIG. 1A.

Next, in some embodiments, a hidden Markov model with a Poisson distribution of read depth may be applied to the percentile values, as shown in step 146. The hidden Markov model may convert the percentile of each bin to one of five CNV statuses: CN=0 (deletion), CN=1 (deletion), CN=2 (normal), CN=3 (duplication) and CN>3 (duplication). Step 146 may correspond to steps 124 and 126 of FIG. 1A.

In some embodiments, where a bin size is set to a small value in step 142 (e.g., 50 base pairs), noise may occur in the assigned CNV statuses. Using larger bin sizes may decrease noise but also may decrease sensitivity to small CNVs. Therefore, merging adjacent CNVs in steps 148, 150, 152, 154, and 156 may mitigate noise in CNV statuses, according to some embodiments described herein. Steps 148, 150, 152, 154, and 156 may correspond to some or all of step 128 of FIG. 1A. In step 148, if a CNV status' length is shorter than 5Kb, the status may be merged with a neighboring status.

In some instances, the CNV status merging may merge regions including too many different statuses, as shown in step 150. To prevent this, if the original status of the region is assigned to less than 80% of the length of the merged region of the sequence, the CNV status merging will stop and reinstate the original statuses and genetic regions, as shown in step 152. After recognition of a complex region and the cease of merging, the CNV statuses may then sorted by their respective sequence lengths, as shown in step 154. From the longest to the shortest, each CNV status may scan other statuses downstream and upstream for further merging, as shown in step 156. An additional step of applying a clustering algorithm, as described in connection with FIG. 1B may be applied during merging of the CNV statuses.

Candidate CNVs may then be generated by filtering the CNV statuses in step 158, in accordance with some embodiments described herein. Step 158 may correspond with some or all of step 130 of FIG. 1A. Each CNV status region may be divided into ten bins of equal length. Each bin may be assigned a uniqueness value corresponding to number of k-mers in the bin which are unique (e.g., only appear once within the genetic sequence). The bins may be sequentially filtered if their uniqueness values are below a threshold value (e.g., if the percentage of unique k-mers is below 60%, though any suitable threshold may be used).

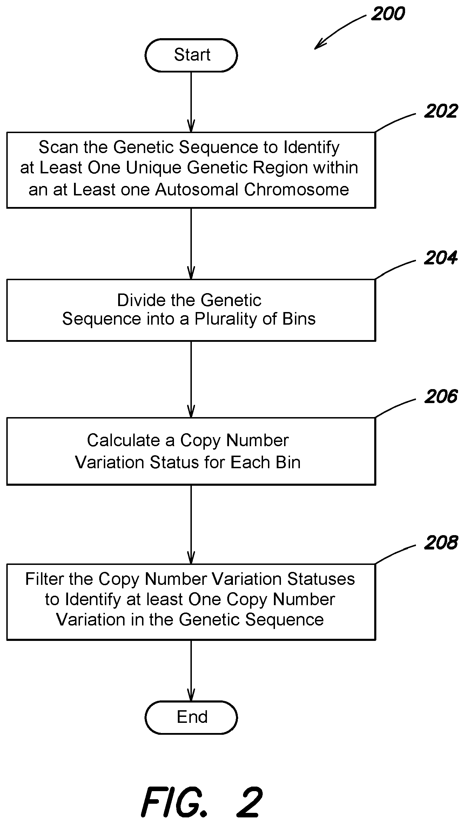

FIG. 2 is a flowchart describing a process 200 of identifying at least one CNV in a genetic sequence, in accordance with some embodiments of the technology described herein. In some embodiments, part or all of the process 200 may be implemented by hardware (e.g., using an ASIC, an FPGA, or any other suitable circuitry), software (e.g., by executing the software using a computer processor), or any suitable combination thereof.

In step 202, the genetic sequence to be analyzed may be scanned to identify at least one unique genetic region within an at least one autosomal chromosome, in accordance with some embodiments described herein. Step 202 may correspond to step 120 as described in connection with FIG. 1A. A genetic region may be considered unique when each k-mer within the region appears only once and the size of the region is larger than 20 Kb (e.g., 20,000 base pairs).

In step 204, the genetic sequence may be divided into a plurality of bins, in accordance with some embodiments described herein. In some embodiments, the bins may comprise 50 base pairs. In some embodiments, the bins may comprise 25 base pairs, 50 base pairs, or 100 base pairs. In some embodiments, where a bin size is set to a small value (e.g., 50 base pairs), noise may occur in assigning CNV statuses in later steps. Using larger bin sizes may decrease noise but also may decrease sensitivity to small CNVs. The choice of bin size may depend on desired sensitivity versus acceptable noise levels.

In step 206, a CNV status may be calculated for each bin, in accordance with some embodiments described herein. Step 206 may correspond to steps 124 and 126 as described in connection with FIG. 1A and/or with step 146 as described in connection with FIG. 1C. A hidden Markov model (HMM) with a Poisson distribution of read depth may be applied to a percentile representation of read depth values of each bin, in accordance with some embodiments described herein. The hidden Markov model may convert the percentile of each bin to one of five CNV statuses: CN=0 (deletion), CN=1 (deletion), CN=2 (normal), CN=3 (duplication) and CN>3 (duplication).

In step 208, the CNV statuses may be filtered to identify at least one CNV in the genetic sequence, in accordance with some embodiments described herein. Step 208 may correspond to step 130 as described in connection with FIG. 1A and/or with step 158 as described in connection with FIG. 1C. Each CNV status region may be divided into ten bins of equal length. Each bin may be assigned a uniqueness value corresponding to number of k-mers in the bin which are unique (e.g., only appear once within the genetic sequence). The bins may be sequentially filtered if their uniqueness values are below a threshold value (e.g., if the percentage of unique k-mers is below 60%, though any suitable threshold may be used). Candidate CNVs may then be generated based on the filtered CNV statuses.

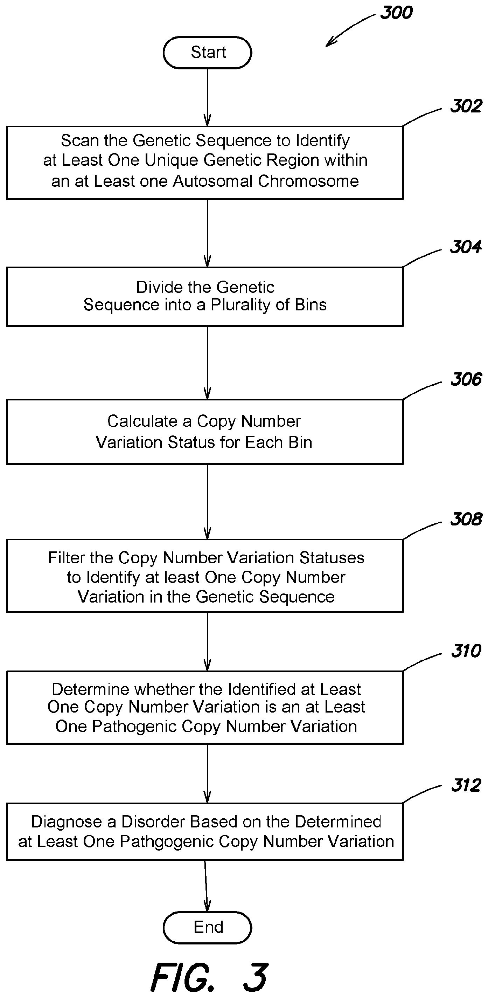

FIG. 3 is a flowchart describing a process 300 of diagnosing a disorder caused by at least one CNV in a genetic sequence, in accordance with some embodiments of the technology described herein. In some embodiments, part or all of the process 300 may be implemented by hardware (e.g., using an ASIC, an FPGA, or any other suitable circuitry), software (e.g., by executing the software using a computer processor), or any suitable combination thereof.

In step 302, the genetic sequence to be analyzed may be scanned to identify at least one unique genetic region within an at least one autosomal chromosome, in accordance with some embodiments described herein. Step 302 may correspond to step 120 as described in connection with FIG. 1A and/or step 202 as described in connection with FIG. 2. A genetic region may be considered unique when each k-mer within the region appears only once and the size of the region is larger than 20 Kb (e.g., 20,000 base pairs).

In step 304, the genetic sequence may be divided into a plurality of bins, in accordance with some embodiments described herein. Step 304 may correspond to step 204 as described in connection with FIG. 2. In some embodiments, the bins may comprise 50 base pairs. In some embodiments, the bins may comprise 25 base pairs, 50 base pairs, or 100 base pairs. In some embodiments, where a bin size is set to a small value (e.g., 50 base pairs), noise may occur in assigning CNV statuses in later steps. Using larger bin sizes may decrease noise but also may decrease sensitivity to small CNVs. The choice of bin size may depend on desired sensitivity versus acceptable noise levels.

In step 306, a CNV status may be calculated for each bin, in accordance with some embodiments described herein. Step 306 may correspond to steps 124 and 126 as described in connection with FIG. 1A, step 146 as described in connection with FIG. 1C, and/or step 206 as described in FIG. 2. A hidden Markov model (HMM) with a Poisson distribution of read depth may be applied to a percentile representation of read depth values of each bin, in accordance with some embodiments described herein. The hidden Markov model may convert the percentile of each bin to one of five CNV statuses: CN=0 (deletion), CN=1 (deletion), CN=2 (normal), CN=3 (duplication) and CN>3 (duplication).

In step 308, the CNV statuses may be filtered to identify at least one CNV in the genetic sequence, in accordance with some embodiments described herein. Step 308 may correspond to step 130 as described in connection with FIG. 1A, with step 158 as described in connection with FIG. 1C, and/or with step 208 as described in connection with FIG. 2. Each CNV status region may be divided into ten bins of equal length. Each bin may be assigned a uniqueness value corresponding to number of k-mers in the bin which are unique (e.g., only appear once within the genetic sequence). The bins may be sequentially filtered if their uniqueness values are below a threshold value (e.g., if the percentage of unique k-mers is below 60%, though any suitable threshold may be used). Candidate CNVs may then be generated based on the filtered CNV statuses.

In step 310, it may be determined whether the identified candidate CNVs include pathogenic CNVs, in accordance with some embodiments described herein. A pathogenic CNV may comprise a CNV which overlaps genomic coordinates for well-known duplication and/or deletion disorders or is otherwise well-documented in the art. Pathogenic CNVs may be, for example, associated with disorders such as, but not limited to, autism-spectrum disorders, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

In some embodiments, determining whether the identified candidate CNVs consist of pathogenic CNVs may comprise a manual review process of the candidate CNVs output by JAX-CNV. In some embodiments, determining whether the identified candidate CNVs include pathogenic CNVs may be a partially or completely automated process using a computing system (e.g., the computing system 900 described in connection with FIG. 9).

In step 312, a disorder may be diagnosed based on the determination that the identified candidate CNVs include pathogenic CNVs, in accordance with some embodiments described herein. The disorder may be diagnosed as any one of, for example, autism-spectrum disorders, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

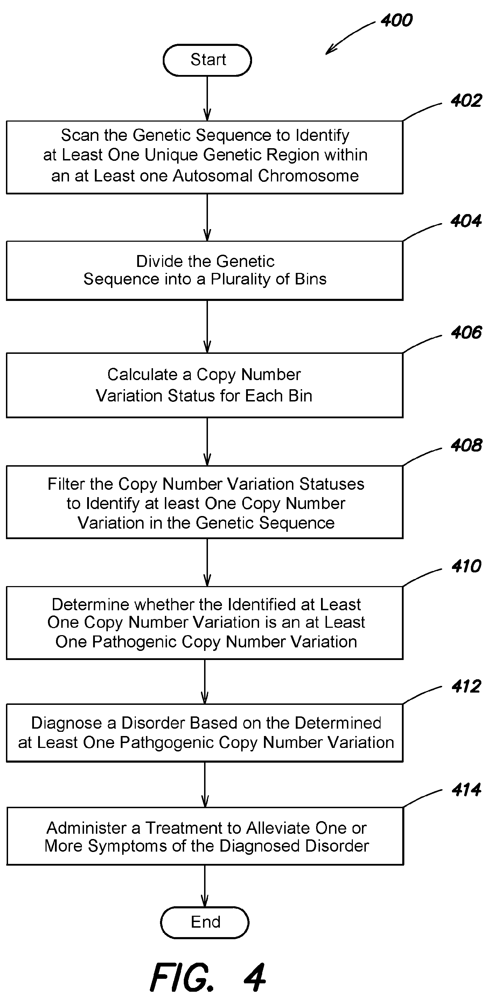

FIG. 4 is a flowchart describing a process 400 of treating a disorder caused by at least one CNV in a genetic sequence, in accordance with some embodiments of the technology described herein. In some embodiments, part or all of the process 400 may be implemented by hardware (e.g., using an ASIC, an FPGA, or any other suitable circuitry), software (e.g., by executing the software using a computer processor), or any suitable combination thereof.

In step 402, the genetic sequence to be analyzed may be scanned to identify at least one unique genetic region within an at least one autosomal chromosome, in accordance with some embodiments described herein. Step 402 may correspond to step 120 as described in connection with FIG. 1A, step 202 as described in connection with FIG. 2, and/or step 302 as described in connection with FIG. 3. A genetic region may be considered unique when each k-mer within the region appears only once and the size of the region is larger than 20 Kb (e.g., 20,000 base pairs).

In step 404, the genetic sequence may be divided into a plurality of bins, in accordance with some embodiments described herein. Step 404 may correspond to step 204 as described in connection with FIG. 2 and/or with step 304 as described in connection with FIG. 3. In some embodiments, the bins may comprise 50 base pairs. In some embodiments, the bins may comprise 25 base pairs, 50 base pairs, or 100 base pairs. In some embodiments, where a bin size is set to a small value (e.g., 50 base pairs), noise may occur in assigning CNV statuses in later steps. Using larger bin sizes may decrease noise but also may decrease sensitivity to small CNVs. The choice of bin size may depend on desired sensitivity versus acceptable noise levels.

In step 406, a CNV status may be calculated for each bin, in accordance with some embodiments described herein. Step 406 may correspond to steps 124 and 126 as described in connection with FIG. 1A, step 146 as described in connection with FIG. 1C, step 206 as described in connection with FIG. 2, and/or step 306 as described in connection with FIG. 3. A hidden Markov model (HMM) with a Poisson distribution of read depth may be applied to a percentile representation of read depth values of each bin, in accordance with some embodiments described herein. The hidden Markov model may convert the percentile of each bin to one of five CNV statuses: CN=0 (deletion), CN=1 (deletion), CN=2 (normal), CN=3 (duplication) and CN>3 (duplication).

In step 408, the CNV statuses may be filtered to identify at least one CNV in the genetic sequence, in accordance with some embodiments described herein. Step 408 may correspond to step 130 as described in connection with FIG. 1A, with step 158 as described in connection with FIG. 1C, step 208 as described in connection with FIG. 2, and/or step 308 as described in connection with FIG. 3. Each CNV status region may be divided into ten bins of equal length. Each bin may be assigned a uniqueness value corresponding to number of k-mers in the bin which are unique (e.g., only appear once within the genetic sequence). The bins may be sequentially filtered if their uniqueness values are below a threshold value (e.g., if the percentage of unique k-mers is below 60%, though any suitable threshold may be used). Candidate CNVs may then be generated based on the filtered CNV statuses.

In step 410, it may be determined whether the identified candidate CNVs include pathogenic CNVs, in accordance with some embodiments described herein. Step 410 may correspond to step 310 as described in connection with FIG. 3. A pathogenic CNV may comprise a CNV which overlaps genomic coordinates for well-known duplication and/or deletion disorders or is otherwise well-documented in the art. Pathogenic CNVs may be, for example, associated with disorders such as, but not limited to, autism-spectrum disorders, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

In some embodiments, determining whether the identified candidate CNVs consist of pathogenic CNVs may comprise a manual review process of the candidate CNVs output by JAX-CNV. In some embodiments, determining whether the identified candidate CNVs consist of pathogenic CNVs may be a partially or completely automated process using a computing system (e.g., the computing system 900 described in connection with FIG. 9).

In step 412, a disorder may be diagnosed based on the determination that the identified candidate CNVs consist of pathogenic CNVs, in accordance with some embodiments described herein. Step 412 may correspond with step 312 as described in connection with FIG. 3. The disorder may be diagnosed as any one of, for example, autism-spectrum disorders, epilepsy, Schizophrenia, TAR syndrome, HNPP syndrome, 3q29 microdeletion syndrome, Sotos syndrome, 8p23.1 deletion syndrome, Langer-Giedion syndrome, WAGR syndrome, Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, 18p deletion syndrome, Cri-du-chat syndrome, Smith-Magenis syndrome, 1p deletion syndrome, Prader-Willi syndrome, De Grouchy syndrome, Xp11.2 duplication syndrome, and Wolf-Hirschhorn syndrome.

In step 414, a treatment may be administered to alleviate one or more symptoms associated with the diagnosed disorder of step 412, in accordance with some embodiments described herein. Treatments may include one or more of genetic counseling, occupational therapy, speech therapy, physical therapy, and/or cardiovascular medicines or surgery.

The inventors have further recognized and appreciated that conventional methods of CNV detection have met certain clinical benchmarks. Accordingly, the inventors have tested JAX-CNV for accuracy and sensitivity across 31 samples associated with various constitutional disorders (i.e., DiGeorge, Williams, Cri-du-chat, Smith-Magenis, Wolf-Hirschhorn, Miller-Dieker Lissencephaly, Tetralogy of fallot, 1p deletion, and Angelman syndromes) from the Coriell Institute (as shown in Table 1). In total, there are 45 CNVs present in the test samples (25 deletions and 20 duplications, ranging from 101 kilobases (Kb) to 94 megabases (Mb) in size) reported by the Coriell Institute, which set an initial baseline for sensitivity analysis of JAX-CNV.

41 of the 45 Coriell registered CNVs were identified as pathogenic. WGS was performed on these samples by Illumina paired-end sequencing with read length 2x150bp and a read depth of approximately 40. BWA-MEM was applied for alignment against the GRCh38 human reference genome (chr1-22, X, Y, and M) followed by JAX-CNV for CNV calling. JAX-CNV accurately detected all 45 Coriell registered CNVs from the WGS data as described in Table 1, where an 'O' denotes CNVs detected by the methods and at different read depths. An '*' denotes that the CNVs were not 50% reciprocally overlapping between detection methods, but were recovered in manual review. Shadowed cells indicate that the CNV was not called.

These 31 test samples were further assessed by a clinically-validated Affymetrix CytoScan HD platform (Affymetrix, Santa Clara, CA) for detection of chromosomal imbalances following the standard operating procedures of the CLIA-certified laboratory at The Jackson Laboratory (herein, "JAX-GM"). The clinical laboratory at JAX-GM, like some other clinical laboratories, provides a higher resolution for clinical CNV detection (i.e., down to 50 Kb) using CMAs. CNV microarray analysis was performed by the Cytogenetics Laboratory at JAX-GM using the Affymetrix Cytoscan HD platform. The array includes 2,696,550 probes that include 743,304 SNP probes and 1,953,246 nonpolymorphic copy-number probes. The average probe spacing for RefSeq genes is 880 bp, and 96% of genes are represented. DNA labeling, slide hybridization, washing, and scanning were performed following the manufacturer's protocol. CEL files were generated from scanned array image files by Affymetrix GeneChip Command Console software and were imported into Affymetrix Chromosome Analysis Suite (ChAS v3.3) software. Copy number data files (CYCHP files) were generated using Affymetrix CytoScan HD Array version NA36 (hg38) as a reference. Data were analyzed using the following filtering criteria: greater than 50Kb with a minimum of 50 consecutive markers.

JAX-GM clinically-validated CMA platform reported a total of 105 CNVs (0-9 CNVs for each sample). The CMA platform failed to detect six Coriell registered CNVs, including four deletions (101.5 Kb - 119 Kb) and two duplications (118 Kb - 148.8 Kb) due to limited probe coverage on the array (Table 1) since at least 50 array probes are required to ensure a reliable and high-quality CNV call by the CMA platform. As a result, JAX-CNV was able to identify all 45 Coriell reported chromosomal aberrations while JAX-GM CMA missed six of them (a 13.33% false negative rate for the JAX-GM CMA platform).

**Table 1**

| **Coriell IDs** | **Coriell Description** | **Coriell CNV** | **CNV Type** | **Pathogenic Annotation** | **JAX-GM CMA** | **JAX-CNV** | | | | | |
|---|---|---|---|---|---|---|---|---|---|---|---|
| | | | | | | **Original_ coverage (42-46x)** | **30x** | **20x** | **15x** | **10x** | **9x** |
| GM02820 | Chromosome aberration | 9p24.3p13. 3 (34.5 Mb) | DUP | G/M | O | O | O | O | O | O | O |
| | | 12q24.32 q24.33 (7.3 Mb) | DEL | G/M | O | O | O | O | O | O | O |
| GM03997 | Derivative chromosome | 5q35.1 (130 Kb) | DUP | M | O | O | O | O | O | O | |
| | | 12p13.33p1 2.2 (20.8 Mb) | DUP | G/D/M | O | O | O | O | O | O | O |
| | | 12q24.33 (623 Kb) | DEL | G/M | O | O | O | O | O | O | O |
| GM05876 | DiGeorge Syndrome | 22q11.21 (1.4 Mb) | DEL | G/D/M | O | O | O | O | O | O | O |
| GM09025 | Ring chromosome | 16q24.2 (383 Kb) | DUP | G/M | O | O | O | O | O | O | * |
| | | 22q13.31q1 3.33 (2.9 Mb) | DEL | G/D/M | O | O | O | O | O | O | O |
| GM09209 | Miller-Dicker lissencephaly Syndrome | 17p13.3 (5.9 Mb) | DEL | G/D/M | O | O | O | O | O | O | O |
| GM09687 | Recombinant chromosome | 16p13.3 (1.1 Mb) | DEL | G/D/M | O | O | O | O | O | O | O |
| | | 16q22.1q24 .3 (20 Mb) | DUP | G/M | O | O | O | O | O | O | O |
| GM09711 | Dicentric chromosome | 2q13 (140 Kb) | DUP | G/M | O | O | O | O | O | * | |
| | | 13q11q34 (94 Mb) | DUP | G/M | O | O | O | O | O | O | O |
| | | 13q34 (1.7 Mb) | DEL | M | O | O | O | O | O | O | O |
| GM10946 | Recombinant chromosome | 6p21.2p21. 1 (964 Kb) | DUP | G/M | O | O | O | O | O | O | O |
| | | 6p12.3 (780 Kb) | DUP | | O | O | O | O | O | O | O |
| | | 6q14.1q16. 3 (25 Mb) | DEL | G/M | O | O | O | O | O | O | O |
| GM11428 | Duplicated chromosome | 3p26.3p26. 2 (5.3 Mb) | DEL | G/M | O | O | O | O | O | O | O |
| | | 3q22.1q26. 1 (29.8 Mb) | DUP | G/D/M | * | O | O | O | O | O | O |
| | | 3q26.1 (112.8 Kb) | DEL | | | O | O | O | O | O | O |
| | | 3q26.1q29 (35.2 Mb) | DUP | G/M | O | O | O | O | O | O | O |
| GM11516 | Angelman Syndrome | 15q11.2q13 .1 (7 Mb) | DEL | G/D/M | O | O | O | O | O | O | O |
| GM13480 | Williams syndrome | 7q11.23 (1.6 Mb) | DEL | G/D/M | O | O | O | O | O | O | O |
| | | 9p24.1 (107.6 Kb) | DUP | | O | O | O | O | O | O | |
| GM13590 | Duplicated chromosome | 2q11.2q21. 1 (33.6 Mb) | DUP | G/M | O | O | O | O | O | O | O |
| | | 2q37.3 (119 Kb) | DEL | | | O | O | O | O | O | O |
| | | 4q31.22 (101.5 Kb) | DEL | M | | O | O | O | O | O | O |
| | | 9p13.3 (120.3 Kb) | DUP | G/M | O | O | O | O | * | * | |
| | | 17q11.1 (101 Kb) | DUP | M | O | O | O | O | O | O | |
| GM13946 | Williams Syndrome | 7q11.23q11 .23 (1.6 Mb) | DEL | G/D/M | O | O | O | O | O | O | O |
| GM14164 | Tetralogy of fallot | 13q14.2 (47.9 Mb) | DEL | G/M | O | O | O | O | O | O | O |
| | | 22q11.21 (148.8 Kb) | DUP | M | | O | O | O | O | | |
| GM16580 | 18p deletion syndrome | 18p11.32 (1.6 Mb) | DEL | M | O | O | O | O | O | O | O |
| | | 18q21.33q2 3 (13.5 Mb) | DUP | M | O | O | O | O | O | O | O |
| | | 18q23 (4.0 Mb) | DEL | G/M | O | O | O | O | O | O | O |
| GM16593 | Cri-du-chat syndrome | 5p15.3 (14.7 Mb) | DEL | G/M | O | O | O | O | O | O | O |
| | | 14q24.3 (2.7 Mb) | DEL | M | O | O | O | O | O | O | O |
| GM18828 | Chromosome aberration | 1q31.3 (118 Kb) | DUP | G/M | | O | O | O | O | | |
| | | 4p16.1 (140 Kb) | DUP | M | O | O | O | O | O | O | O |
| GM20200 | Isodicentric chromosome | 1q31.3 (103 Kb) | DEL | G/M | | O | O | O | O | O | O |
| | | 15q11.1q13 .1 (8.5 Mb) | DUP | G/D/M | O | O | O | O | O | O | O |
| GM20375 | Angelman Syndrome | 15q11.2q13 .1 (4.9 Mb) | DEL | G/D/M | O | O | O | O | O | O | O |
| GM20743 | Smith-Magenis syndrome | 17p11.2 (2.1 Mb) | DEL | G/D/M | O | O | O | O | O | O | O |
| GM22569 | 1p deletion syndrome | 1p36.33 (5.5 Mb) | DEL | G/M | O | O | O | O | O | O | O |
| GM22601 | Wolf-I Irachhorm syndrome | 4p16.3 (25.0 Mb) | DEL | G/D/M | O | O | O | O | O | O | O |

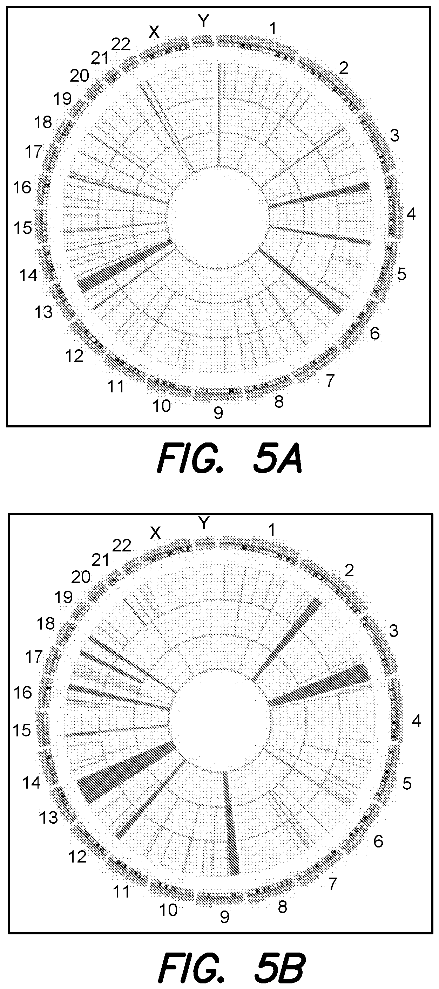

FIGs. 5A and 5B show a summary of Table 1, comparing of detected CNV deletions (FIG. 5A) and duplications (FIG. 5B) for 31 samples as identified by a CMA performed by the Coriell Institute, a CMA performed by The Jackson Laboratory, and whole genome sequences (WGSs) as analyzed by the JAX-CNV algorithm, in accordance with some embodiments of the technology described herein. The CMA performed by the Coriell Institute is represented by the inner circle, the CMA performed by the JAX-GM is represented by the middle circle, and the analysis performed by JAX-CNV is represented by the outer circle, with divisions representing individual chromosomes arranged around the circumference of the circles.

Since the Affymetrix CytoScan HD is a clinically-validated platform at JAX-GM, all CNVs identified by this platform should ideally be detected by JAX-CNV to show the potential of WGS with JAX-CNV as a first-tier diagnostic assay. The CNV size cutoff of the CMA platform at JAX-GM is ≧ 50 Kb. By this criterion, the JAX-GM CMA platform identified 112 CNVs from the 31 test samples, including 39 of the 45 Coriell registered CNVs. Among the 112 CNVs, four deletions and three duplications were marginal quality calls, and were therefore, subsequently validated by ddPCR assay. ddPCR assays for these seven regions were designed, except for a 69 Kb gain at 16p13 (chr16:14961449-15030399) due to the complexity of that genomic region.

The ddPCR reactions were created following the Bio-Rad QX200^{™} system manufacturer protocol. A total of 10ng DNA template was mixed with a 2X ddPCR SuperMix for Probes (no dUTP), HindIII-HF enzyme (2U/reaction) (New England BioLabs, MA, USA), 20X primer/probe, (both FAM and HEX-labeled probes) and water to a final volume of 20 µL. Each reaction mixture was then loaded into the sample well of an eight-channel droplet generator cartridge. A volume of 70 µl of droplet generation oil was loaded into the oil well for each channel and covered with a gasket. The cartridge was placed into the Bio-Rad QX200^{™} Droplet Generator. After the droplets were generated in the droplet well, 40 µl was transferred into a 96-well PCR plate and then heat-sealed with a foil seal. PCR amplification was performed using a C1000 Touch thermal cycler with the following conditions for CNV detection: enzyme activation at 95°C for 10 minutes, denaturation and extension at 94°C for 30 seconds and 60°C for 1 minute for a total of 40 cycles, enzyme deactivation at 98°C for 10 minutes, finished with a 4°C hold. Once completed, the 96-well PCR plate was loaded on the QX200^{™} Droplet Reader. All experiments had at least two normal controls, and a no-template control (NTC) with water. All samples and controls were run in duplicate, and data from any well with less than 8,000 droplets was treated as failed QC and excluded for downstream analysis. Analysis of the ddPCR data utilized QuantaSoft^{™} software.

The remaining six aberrations (four deletions and two duplications) were confirmed by ddPCR to be false positives by the CMA platform. The most interesting falsepositive CNV was a deletion at 6p25 that is located in a commonly duplicated region. The 1000 Genomes Project3,25 including 2,504 samples showed a 0.99 allele frequency of this duplication in the 26 studied populations. Therefore, this "deletion" could actually be a normal two copy number result but appears as a deletion because reference samples carry a duplication. Consequently, 105 CNVs (61 deletions and 44 duplications) were used for the comparison with JAX-CNV described below.

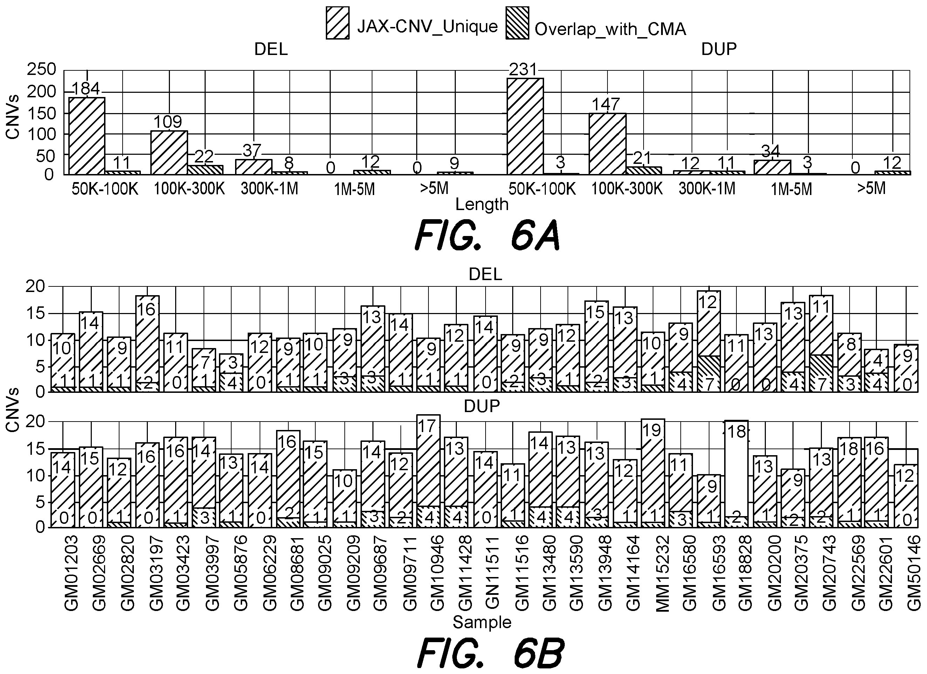

JAX-CNV successfully identified all 105 CNVs (65 were identified as pathogenic) from WGS data (Figure 3) when a 50% reciprocal overlap was applied to evaluate the CNV calls. Of note, there were two deletions (GM11428 and GM14164) and four duplications (GM03997, GM09687, GM11428 and GM13590) that did not meet the benchmark of 50% reciprocal overlap with the CMA calls, but they were still located in the same regions with either smaller or larger size ones. FIG. 6A shows, as a function of CNV size and for both CNV deletions and CNV duplications, the number of unique CNVs (light grey) detected by JAX-CNV and the number of CNVs detected by both JAX-CNV and the CMAs performed by The Jackson Laboratory (dark grey) on the 31 samples described in Table 1, in accordance with some embodiments of the technology described herein. FIG. 6B shows, for each genetic mutation, the number of unique CNVs (light grey) detected by JAX-CNV and the number of CNVs detected by both JAX-CNV and CMAs performed by The Jackson Laboratory (dark grey) on 31 samples described in Table 1, in accordance with some embodiments of the technology described herein. Overall, JAX-CNV detected 754 more CNVs than the CMA performed by JAX-GM, an average of 10 more CNVs for each sample. 280 of the detected CNVs were considered pathogenic. More than half of the JAX-CNV unique calls are smaller than 100 Kb and 89% are smaller than 300 Kb. This may be due to the fact that WGS and JAX-CNV provide higher resolution than array-based technologies, which are limited by the number of probes used.

Although the costs of NGS have dropped, the inventors have recognized and appreciated that its price still remains prohibitive when considering WGS as a first-tier assay in clinical diagnostics. To tackle this issue and demonstrate the ability of JAX-CNV, the inventors have downsampled the read depth of the WGS data and assessed JAX-CNV's sensitivity on these lower read depths, in accordance with some embodiments described herein. These samples were originally sequenced with the read depths ranging from 30x to 48x. The simulation of different coverages was performed by SAMBAMBA35 on the aligned BAM files. A series of read depths including 30x, 20x, 15x, 10x, and 9x were generated based on the original WGS data. JAX-CNV was then applied on the downsampled WGS data with different read depths.

Among the 45 Coriell registered CNVs, 33 were larger than 300 Kb, which is the CAP standard cutoff size. Even when the read depth was reduced to 9x, JAX-CNV remained 100% sensitive for the detection of these CNVs greater than 300 Kb. The use of a read depth of 9x may significantly reduce the cost of WGS for clinical diagnosis.

For the remaining 12 CNVs smaller than 300 Kb, JAX-CNV obtained reproducible results for sequencing read depth down to 15x, or 31.25-50% of the original read depths (see Table 1). At a sequencing read depth of 10x, JAX-CNV failed to identify two duplications, one 148.8 Kb duplication at chromosome region 22q11.21 of GM14164, and another 118 Kb duplication at chromosome region 1q31 of GM18828. Both duplications were also not detected by the JAX-GM CMA. At a read depth of 9x, JAX-CNV identified all deletions, including four calls that JAX-GM CMA failed to identify; however, JAX-CNV missed seven duplications, including a 130 Kb duplication at chromosome region 5q35 of GM03997, 140 Kb duplication at chromosome region 2q13 of GM09711, 107 Kb duplication at chromosome region 9p24 of GM13480, 120 Kb duplication at chromosome region 9q13 of GM13590, 101 Kb duplication at chromosome region 17q11 of GM13590, 148 Kb duplication at chromosome region 22q11 of GM14164, and 118 Kb duplication at chromosome region 1q31 of GM18828.

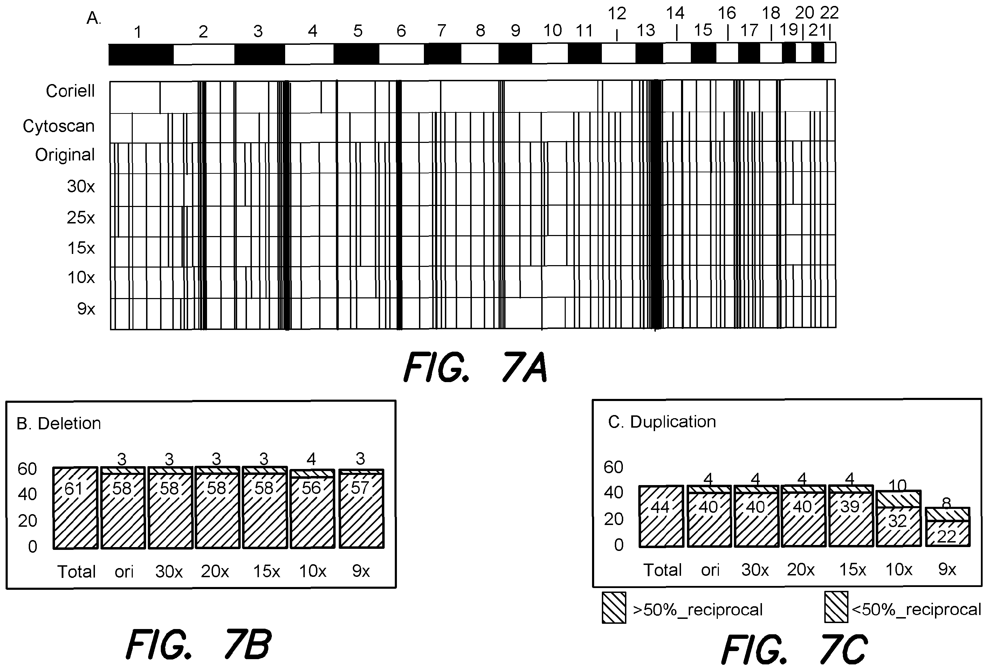

To better understand the effect of sequencing read depths, the inventors extended analysis to the 105 CNVs called by JAX-GM CMA. FIG. 7A shows CNV detection by, from top to bottom and for the 105 CNVs called by the JAX-GM CMA, CMAs performed by the Coriell Institute, CMAs performed by JAX-GM, and analysis of WGSs by JAX-CNV for decreasing values of read depth, in accordance with some embodiments described herein. A 100% concordance was achieved at 20x read depth for all 105 CNVs (61 deletions and 44 duplications). However, as the read depth decreased, the concordance between methods decreased. For 15x, 10x and 9x sequence read depths, respectively, JAX-CNV missed one CNV (duplication), four CNVs (a deletion and three duplications), and 15 CNVs (a deletion and 14 duplications) respectively.

FIG. 7B shows, as a function of coverage and for CNV deletions, concordance between JAX-CNV and CMAs performed by The Jackson Laboratory on 31 samples, in accordance with some embodiments of the technology described herein. FIG. 7C shows, as a function of coverage and for CNV duplications, concordance between JAX-CNV and CMAs performed by The Jackson Laboratory on 31 samples, in accordance with some embodiments of the technology described herein. The lengths of missed CNVs range from 79 Kb to 311 Kb. Thus, the concordance between JAX-GM CMA and JAX-CNV on WGS are 100% for 20x sequence read depth, 99% for 15x sequence read depth, 96% for 10x sequencing read depth, and 87% for 9x sequencing read depth. Deletions (FIG. 7B) exhibited a higher concordance rate than duplications (FIG. 7C) with coverage of 15x or lower.

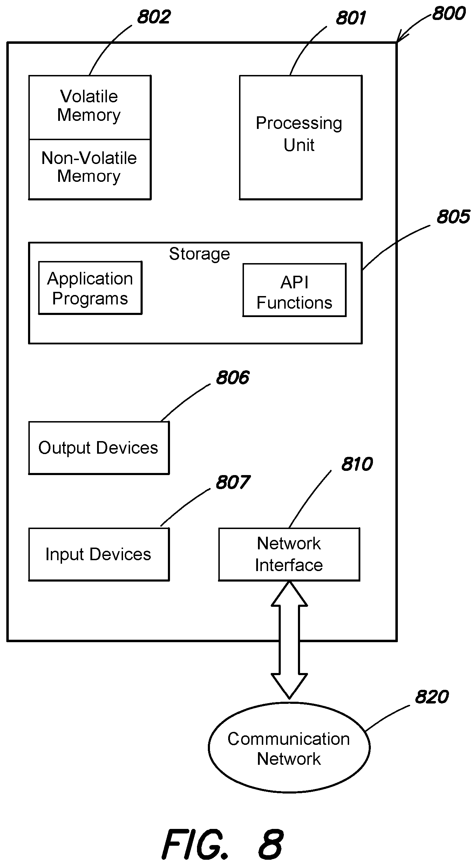

FIG. 8 shows, schematically, an illustrative computer 800 on which any aspect of the present disclosure may be implemented.

In the embodiment shown in FIG. 8, the computer 800 includes a processing unit 801 having one or more processors and a non-transitory computer-readable storage medium 802 that may include, for example, volatile and/or non-volatile memory. The memory 802 may store one or more instructions to program the processing unit 801 to perform any of the functions described herein. The computer 800 may also include other types of non-transitory computer-readable medium, such as storage 805 (e.g., one or more disk drives) in addition to the system memory 802. The storage 805 may also store one or more application programs and/or resources used by application programs (e.g., software libraries), which may be loaded into the memory 1302.

The computer 800 may have one or more input devices and/or output devices, such as devices 806 and 807 illustrated in FIG. 8. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards and pointing devices, such as mice, touch pads, and digitizing tablets. As another example, the input devices 807 may include a microphone for capturing audio signals, and the output devices 806 may include a display screen for visually rendering, and/or a speaker for audibly rendering, recognized text. As another example, the input devices 807 may include sensors (e.g., electrodes in a pacemaker), and the output devices 806 may include a device configured to interpret and/or render signals collected by the sensors (e.g., a device configured to generate an electrocardiogram based on signals collected by the electrodes in the pacemaker).

As shown in FIG. 8, the computer 800 may also comprise one or more network interfaces (e.g., the network interface 810) to enable communication via various networks (e.g., the network 820). Examples of networks include a local area network or a wide area network, such as an enterprise network or the Internet. Such networks may be based on any suitable technology and may operate according to any suitable protocol and may include wireless networks, wired networks or fiber optic networks. Such networks may include analog and/or digital networks.

Having thus described several aspects of at least one embodiment of this technology, it is to be appreciated that various alterations, modifications, and improvements will readily occur to those skilled in the art.

Such alterations, modifications, and improvements are intended to be part of this disclosure. Further, though advantages of the present invention are indicated, it should be appreciated that not every embodiment of the technology described herein will include every described advantage. Some embodiments may not implement any features described as advantageous herein and in some instances one or more of the described features may be implemented to achieve further embodiments. Accordingly, the foregoing description and drawings are by way of example only.

The above-described embodiments of the technology described herein can be implemented in any of numerous ways. For example, the embodiments may be implemented using hardware, software or a combination thereof. When implemented in software, the software code can be executed on any suitable processor or collection of processors, whether provided in a single computer or distributed among multiple computers. Such processors may be implemented as integrated circuits, with one or more processors in an integrated circuit component, including commercially available integrated circuit components known in the art by names such as CPU chips, GPU chips, microprocessor, microcontroller, or co-processor. Alternatively, a processor may be implemented in custom circuitry, such as an ASIC, or semi-custom circuitry resulting from configuring a programmable logic device. As yet a further alternative, a processor may be a portion of a larger circuit or semiconductor device, whether commercially available, semi-custom or custom. As a specific example, some commercially available microprocessors have multiple cores such that one or a subset of those cores may constitute a processor. Though, a processor may be implemented using circuitry in any suitable format.

Also, the various methods or processes outlined herein may be coded as software that is executable on one or more processors running any one of a variety of operating systems or platforms. Such software may be written using any of a number of suitable programming languages and/or programming tools, including scripting languages and/or scripting tools. In some instances, such software may be compiled as executable machine language code or intermediate code that is executed on a framework or virtual machine. Additionally, or alternatively, such software may be interpreted.

The techniques disclosed herein may be embodied as a non-transitory computer-readable medium (or multiple computer-readable media) (e.g., a computer memory, one or more floppy discs, compact discs, optical discs, magnetic tapes, flash memories, circuit configurations in Field Programmable Gate Arrays or other semiconductor devices, or other non-transitory, tangible computer storage medium) encoded with one or more programs that, when executed on one or more processors, perform methods that implement the various embodiments of the present disclosure discussed above. The computer-readable medium or media may be transportable, such that the program or programs stored thereon may be loaded onto one or more different computers or other processors to implement various aspects of the present disclosure as discussed above.

The terms "program" or "software" are used herein to refer to any type of computer code or set of computer-executable instructions that may be employed to program one or more processors to implement various aspects of the present disclosure as discussed above. Moreover, it should be appreciated that according to one aspect of this embodiment, one or more computer programs that, when executed, perform methods of the present disclosure need not reside on a single computer or processor, but may be distributed in a modular fashion amongst a number of different computers or processors to implement various aspects of the present disclosure.

Computer-executable instructions may be in many forms, such as program modules, executed by one or more computers or other devices. Program modules may include routines, programs, objects, components, data structures, etc. that perform particular tasks or implement particular abstract data types. Functionalities of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields to locations in a computer-readable medium that convey relationship between the fields. However, any suitable mechanism may be used to establish a relationship between information in fields of a data structure, including through the use of pointers, tags, or other mechanisms that establish relationship between data elements.

Various aspects of the present invention may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Also, the invention may be embodied as a method, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter well as additional items.

## Claims

1. A method for detecting at least one copy number variation, CNV, in a genetic sequence, the method comprising:
using a processor to perform steps of:
dividing the genetic sequence into a plurality of bins, each bin of the plurality of bins comprising a plurality of base pairs of the genetic sequence;
calculating a CNV status for each bin of the plurality of bins by:
calculating a read depth of each bin of the plurality of bins;
converting the read depth of each bin of the plurality of bins into a percentile; and
converting each percentile into a CNV status, wherein converting the percentile of each bin to a CNV status comprises applying a Hidden Markov Model, HMM, with a Poisson distribution of read depth of the genetic sequence; and
merging bins of the plurality of bins to create merged bins;
identifying the at least one CNV in the genetic sequence by:
dividing the merged bins into a plurality of regions, each region comprising an equal number of base pairs;
assigning a uniqueness value to each region; and
filtering out regions having a uniqueness value below a threshold value; and
identifying the at least one CNV based on one or more regions that remain following the filtering.

2. The method of claim 1, wherein the genetic sequence is a partial genome sequence.

3. The method of claim 1, wherein the genetic sequence is a whole genome sequence, WGS.

4. The method of any one of claims 1-3, further comprising:
aligning the genetic sequence with a reference genome to generate genetic sequence alignment data, wherein:
dividing the genetic sequence into a plurality of bins comprises dividing the genetic sequence alignment data into a plurality of bins.

5. The method of any one of claims 1-4, further comprising:
identifying an at least one unique genetic region within an at least one autosomal chromosome; and
calculating a read depth for the at least one unique genetic region, wherein identifying the at least one unique genetic region within the at least one autosomal chromosome comprises:
determining that each 25 k-mer of the at least one unique genetic regions appears only once within the genetic sequence; and
determining that the at least one unique genetic region comprises greater than 20,000 base pairs.

6. The method of claim 5, further comprising calculating a read depth for the genetic sequence.

7. The method of claim 6, further comprising:
calculating a read depth of the at least one autosomal chromosome based on a read depth of the at least one unique genetic region;
comparing the read depth of the at least one autosomal chromosome to the read depth of the genetic sequence; and
determining whether the genetic sequence comprises an aneuploidy based on the compared read depths.

8. The method of any one of claims 1-7, wherein converting the read depth to a percentile comprises:
dividing the read depth of each bin of the plurality of bins by the number of base pairs in the plurality of base pairs and multiplying by the read depth of the genetic sequence.

9. The method of any one of claims 1-8, wherein each bin of the plurality of bins comprises 50 base pairs.

10. The method of claim 1, wherein the uniqueness value is calculated by determining a number of unique k-mers in the regions.

11. At least one non-transitory computer-readable storage medium, having computer-readable instructions stored thereon that, when executed by a processor, cause the processor to execute a method to detect at least one copy number variation, CNV, in a genetic sequence, the method comprising:
dividing the genetic sequence into a plurality of bins, each bin of the plurality of bins comprising a plurality of base pairs of the genetic sequence;
calculating a CNV status for each bin of the plurality of bins by:
calculating a read depth of each bin of the plurality of bins;
converting the read depth of each bin of the plurality of bins into a percentile; and
converting each percentile into a CNV status, wherein converting the percentile of each bin to a CNV status comprises applying a Hidden Markov Model, HMM, with a Poisson distribution of read depth of the genetic sequence; and
merging bins of the plurality of bins having a same CNV status to create merged bins;
identifying the at least one CNV in the genetic sequence by:
dividing the merged bins into a plurality of regions, each region comprising an equal number of base pairs;
assigning a uniqueness value to each region; and
filtering out regions having a uniqueness value below a threshold value; and identifying the at least one CNV based on one or more regions that remain following the filter.

12. The at least one non-transitory computer-readable storage medium of claim 11, the method further comprising:
calculating a read depth for the genetic sequence;
calculating a read depth of the at least one autosomal chromosome based on a read depth of the at least one unique genetic region;
comparing the read depth of the at least one autosomal chromosome to the read depth of the genetic sequence; and
determining whether the genetic sequence comprises an aneuploidy based on the compared read depths.

13. The at least one non-transitory computer-readable storage medium of any one of claims 11 or 12, wherein converting the read depth to a percentile comprises:
dividing the read depth of each bin of the plurality of bins by the number of base pairs in the plurality of base pairs and multiplying by the read depth of the genetic sequence.

14. The at least one non-transitory computer-readable storage medium of claim 11, 12 or 13, wherein the uniqueness value is calculated by determining a number of unique k-mers in the regions.

## Patentansprüche

1. Verfahren zum Nachweisen mindestens einer Kopienzahlvariation, CNV, in einer genetischen Sequenz, das Verfahren umfassend:
Verwenden eines Prozessors zum Durchführen der Schritte:
Unterteilen der genetischen Sequenz in eine Vielzahl von Bins, wobei jedes Bin der Vielzahl von Bins eine Vielzahl von Basenpaaren der genetischen Sequenz umfasst;
Berechnen eines CNV-Status für jeden Bin der Vielzahl von Bins durch:
Berechnen einer Lesetiefe jedes Bins der Vielzahl von Bins;
Umwandeln der Lesetiefe jedes Bins der Vielzahl von Bins in ein Perzentil; und
Umwandeln jedes Perzentils in einen CNV-Status, wobei das Umwandeln des Perzentils jedes Bins in einen CNV-Status das Anwenden eines Hidden-Markov-Modells, HMM, mit einer Poisson-Verteilung der Lesetiefe der genetischen Sequenz umfasst; und
Zusammenfassen von Bins der Vielzahl von Bins, um zusammengefasste Bins zu erzeugen;
Identifizieren der mindestens einen CNV in der genetischen Sequenz durch:
Unterteilen der zusammengefassten Bins in eine Vielzahl von Regionen, wobei jede Region eine gleiche Anzahl von Basenpaaren umfasst;
Zuweisen eines Einzigartigkeitswerts zu jeder Region; und
Herausfiltern von Regionen mit einem Eindeutigkeitswert unter einem Grenzwert;
und
Identifizieren der mindestens einen CNV basierend auf einer oder mehreren Regionen, die nach dem Filtern verbleiben.

2. Verfahren nach Anspruch 1, wobei die genetische Sequenz eine Teilgenomsequenz ist.

3. Verfahren nach Anspruch 1, wobei die genetische Sequenz eine Gesamtgenomsequenz, WGS, ist.

4. Verfahren nach einem der Ansprüche 1 bis 3, ferner umfassend:
Ausrichten der genetischen Sequenz mit einem Referenzgenom, um genetische Sequenzausrichtungsdaten zu erzeugen, wobei:
das Unterteilen der genetischen Sequenz in eine Vielzahl von Bins das Unterteilen der Ausrichtungsdaten der genetischen Sequenz in eine Vielzahl von Bins umfasst.

5. Verfahren nach einem der Ansprüche 1 bis 4, ferner umfassend:
Identifizieren mindestens einer einzigartigen genetischen Region innerhalb mindestens eines autosomalen Chromosoms; und
Berechnen einer Lesetiefe für die mindestens eine einzigartige genetische Region, wobei das Identifizieren der mindestens einen einzigartigen genetischen Region innerhalb des mindestens einen autosomalen Chromosoms Folgendes umfasst:
Bestimmen, dass jedes 25 k-mer der mindestens einen einzigartigen genetischen Region nur einmal innerhalb der genetischen Sequenz vorkommt; und
Bestimmen, dass die mindestens eine einzigartige genetische Region mehr als 20.000 Basenpaare umfasst.

6. Verfahren nach Anspruch 5, ferner umfassend das Berechnen einer Lesetiefe für die genetische Sequenz.

7. Verfahren nach Anspruch 6, ferner umfassend:
Berechnen einer Lesetiefe des mindestens einen autosomalen Chromosoms basierend auf einer Lesetiefe der mindestens einen einzigartigen genetischen Region;
Vergleichen der Lesetiefe des mindestens einen autosomalen Chromosoms mit der Lesetiefe der genetischen Sequenz; und
Bestimmen, ob die genetische Sequenz eine Aneuploidie umfasst, basierend auf den verglichenen Lesetiefen.

8. Verfahren nach einem der Ansprüche 1 bis 7, wobei das Umwandeln der Lesetiefe in ein Perzentil Folgendes umfasst:
Unterteilen der Lesetiefe jedes Bins der Vielzahl von Bins durch die Anzahl von Basenpaaren in der Vielzahl von Basenpaaren und Multiplizieren mit der Lesetiefe der genetischen Sequenz.

9. Verfahren nach einem der Ansprüche 1 bis 8, wobei jeder Bin der Vielzahl von Bins 50 Basenpaare umfasst.

10. Verfahren nach Anspruch 1, wobei der Eindeutigkeitswert durch Bestimmen einer Anzahl von eindeutigen k-Meren in den Regionen berechnet wird.

11. Mindestens ein nichtflüchtiges computerlesbares Speichermedium mit darauf gespeicherten computerlesbaren Anweisungen, die, wenn sie von einem Prozessor ausgeführt werden, den Prozessor veranlassen, ein Verfahren zum Nachweisen mindestens einer Kopienzahlvariation, CNV, in einer genetischen Sequenz auszuführen, das Verfahren umfassend:
Unterteilen der genetischen Sequenz in eine Vielzahl von Bins, wobei jedes Bin der Vielzahl von Bins eine Vielzahl von Basenpaaren der genetischen Sequenz umfasst;
Berechnen eines CNV-Status für jeden Bin der Vielzahl von Bins durch:
Berechnen einer Lesetiefe jedes Bins der Vielzahl von Bins;
Umwandeln der Lesetiefe jedes Bins der Vielzahl von Bins in ein Perzentil; und
Umwandeln jedes Perzentils in einen CNV-Status, wobei das Umwandeln des Perzentils jedes Bins in einen CNV-Status das Anwenden eines Hidden-Markov-Modells, HMM, mit einer Poisson-Verteilung der Lesetiefe der genetischen Sequenz umfasst; und
Zusammenfassen von Bins der Vielzahl von Bins, die einen gleichen CNV-Status aufweisen, um zusammengefasste Bins zu erzeugen; Identifizieren der mindestens einen CNV in der genetischen Sequenz durch:
Unterteilen der zusammengefassten Bins in eine Vielzahl von Regionen, wobei jede Region eine gleiche Anzahl von Basenpaaren umfasst;
Zuweisen eines Einzigartigkeitswerts zu jeder Region; und
Herausfiltern von Regionen mit einem Eindeutigkeitswert unter einem Grenzwert; und Identifizieren der mindestens einen CNV basierend auf einer oder mehreren Regionen, die nach dem Filter verbleiben.

12. Mindestens ein nichtflüchtiges computerlesbares Speichermedium nach Anspruch 11, das Verfahren ferner umfassend:
Berechnen einer Lesetiefe für die genetische Sequenz;
Berechnen einer Lesetiefe des mindestens einen autosomalen Chromosoms basierend auf einer Lesetiefe der mindestens einen einzigartigen genetischen Region;
Vergleichen der Lesetiefe des mindestens einen autosomalen Chromosoms mit der Lesetiefe der genetischen Sequenz; und
Bestimmen, ob die genetische Sequenz eine Aneuploidie umfasst, basierend auf den verglichenen Lesetiefen.

13. Mindestens eine nichtflüchtige computerlesbare Speichermedium nach einem der Ansprüche 11 oder 12, wobei das Umwandeln der Lesetiefe in ein Perzentil Folgendes umfasst:
Unterteilen der Lesetiefe jedes Bins der Vielzahl von Bins durch die Anzahl von Basenpaaren in der Vielzahl von Basenpaaren und Multiplizieren mit der Lesetiefe der genetischen Sequenz.

14. Mindestens eine nichtflüchtige computerlesbare Speichermedium nach Anspruch 11, 12 oder 13, wobei der Eindeutigkeitswert durch Bestimmen einer Anzahl von eindeutigen k-Meren in den Regionen berechnet wird.

## Revendications

1. Procédé de détection d'au moins une variation du nombre de copies, CNV, dans une séquence génétique, le procédé comprenant :
l'utilisation d'un processeur pour effectuer les étapes consistant à :
diviser la séquence génétique en une pluralité de segments, chaque segment de la pluralité de segments comprenant une pluralité de paires de bases de la séquence génétique ;
calculer un statut de CNV pour chaque compartiment de la pluralité de compartiments par :
le calcul d'une profondeur de lecture de chaque compartiment de la pluralité de compartiments ;
la conversion de la profondeur de lecture de chaque compartiment de la pluralité de compartiments en un percentile ; et
la conversion de chaque centile en un statut de CNV, dans lequel la conversion du centile de chaque segment en un statut de CNV comprend l'application d'un modèle de Markov caché, HMM, avec une distribution de Poisson de la profondeur de lecture de la séquence génétique ; et
la fusion des compartiments de la pluralité de compartiments pour créer des compartiments fusionnés ;
l'identification de l'au moins un CNV dans la séquence génétique par :
la division des segments fusionnés en une pluralité de régions, chaque région comprenant un nombre égal de paires de bases ;
l'attribution d'une valeur d'unicité à chaque région ; et
l'élimination par filtrage des régions ayant une valeur d'unicité inférieure à une valeur de seuil ;
et
l'identification de l'au moins un CNV sur la base d'une ou plusieurs régions qui restent après le filtrage.

2. Procédé selon la revendication 1, dans lequel la séquence génétique est une séquence partielle de génome.

3. Procédé selon la revendication 1, dans lequel la séquence génétique est une séquence de génome entier, WGS.

4. Procédé selon l'une quelconque des revendications 1 à 3, comprenant en outre :
l'alignement de la séquence génétique avec un génome de référence pour générer des données d'alignement de séquence génétique, dans lequel :
la division de la séquence génétique en une pluralité de compartiments comprend la division des données d'alignement de séquence génétique en une pluralité de compartiments.

5. Procédé selon l'une quelconque des revendications 1 à 4, comprenant en outre :
l'identification d'au moins une région génétique unique dans au moins un chromosome autosomique ; et
le calcul d'une profondeur de lecture pour l'au moins une région génétique unique, dans lequel l'identification de l'au moins une région génétique unique dans l'au moins un chromosome autosomique comprend :
la détermination que chaque 25 k-mère de l'au moins une région génétique unique n'apparaît qu'une seule fois dans la séquence génétique ; et
la détermination que l'au moins une région génétique unique comprend plus de 20 000 paires de bases.

6. Procédé selon la revendication 5, comprenant en outre le calcul d'une profondeur de lecture pour la séquence génétique.

7. Procédé selon la revendication 6, comprenant en outre :
le calcul d'une profondeur de lecture de l'au moins un chromosome autosomique sur la base d'une profondeur de lecture de l'au moins une région génétique unique ;
la comparaison de la profondeur de lecture de l'au moins un chromosome autosomique à la profondeur de lecture de la séquence génétique ; et
le fait de déterminer si la séquence génétique comprend une aneuploïdie sur la base des profondeurs de lecture comparées.

8. Procédé selon l'une quelconque des revendications 1 à 7, dans lequel la conversion de la profondeur de lecture en un percentile comprend :
la division de la profondeur de lecture de chaque compartiment de la pluralité de compartiments par le nombre de paires de bases dans la pluralité de paires de bases et multiplier par la profondeur de lecture de la séquence génétique.

9. Procédé selon l'une quelconque des revendications 1 à 8, dans lequel chaque compartiment de la pluralité de compartiments comprend 50 paires de bases.

10. Procédé selon la revendication 1, dans lequel la valeur d'unicité est calculée en déterminant un nombre de k-mères uniques dans les régions.

11. Au moins un support de stockage non volatil lisible par un ordinateur, sur lequel sont stockées des instructions lisibles par un ordinateur qui, lorsqu'elles sont exécutées par un processeur, amènent le processeur à exécuter un procédé pour détecter au moins une variation du nombre de copies, CNV, dans une séquence génétique, le procédé comprenant :
la division de la séquence génétique en une pluralité de segments, chaque segment de la pluralité de segments comprenant une pluralité de paires de bases de la séquence génétique ;
le calcul d'un statut de CNV pour chaque compartiment de la pluralité de compartiments par :
le calcul d'une profondeur de lecture de chaque compartiment de la pluralité de compartiments ;
la conversion de la profondeur de lecture de chaque compartiment de la pluralité de compartiments en un percentile ; et
la conversion de chaque centile en un statut de CNV, dans lequel la conversion du centile de chaque segment en un statut de CNV comprend l'application d'un modèle de Markov caché, HMM, avec une distribution de Poisson de la profondeur de lecture de la séquence génétique ; et
la fusion des segments de la pluralité de segments ayant un même statut de CNV pour créer des segments fusionnés ; l'identification de l'au moins un CNV dans la séquence génétique par :
la division des segments fusionnés en une pluralité de régions, chaque région comprenant un nombre égal de paires de bases ;
l'attribution d'une valeur d'unicité à chaque région ; et
l'élimination par filtrage des régions ayant une valeur d'unicité inférieure à une valeur de seuil ; et l'identification de l'au moins un CNV sur la base d'une ou plusieurs régions qui restent après le filtre.

12. Support ou supports de stockage non transitoires lisibles par ordinateur selon la revendication 11, le procédé comprenant en outre :
le calcul d'une profondeur de lecture pour la séquence génétique ;
le calcul d'une profondeur de lecture de l'au moins un chromosome autosomique sur la base d'une profondeur de lecture de l'au moins une région génétique unique ;
la comparaison de la profondeur de lecture de l'au moins un chromosome autosomique à la profondeur de lecture de la séquence génétique ; et
le fait de déterminer si la séquence génétique comprend une aneuploïdie sur la base des profondeurs de lecture comparées.

13. Support ou supports de stockage non transitoires lisibles par ordinateur selon l'une quelconque des revendications 11 ou 12, dans lesquels la conversion de la profondeur de lecture en un centile comprend :
la division de la profondeur de lecture de chaque compartiment de la pluralité de compartiments par le nombre de paires de bases dans la pluralité de paires de bases et multiplier par la profondeur de lecture de la séquence génétique.

14. Support ou supports de stockage non transitoires lisibles par ordinateur selon la revendication 11, 12 ou 13, dans lesquels la valeur d'unicité est calculée en déterminant un nombre de k-mères uniques dans les régions.
